(19) Europäisches Patentamt
European Patent Office
Office européen des brevets

(11) EP 1 037 938 B1

(12) # FASCICULE DE BREVET EUROPEEN

(45) Date de publication et mention de la délivrance du brevet:
**25.08.2004 Bulletin 2004/35**

(21) Numéro de dépôt: **98956957.9**

(22) Date de dépôt: **26.11.1998**

(51) Int Cl.[7]: **C08G 83/00**, A61K 7/48

(86) Numéro de dépôt international:
**PCT/FR1998/002538**

(87) Numéro de publication internationale:
**WO 1999/032540 (01.07.1999 Gazette 1999/26)**

(54) **Utilisation de polymères hyperbranchés ou dendrimères ayant un groupement particulier en tant qu'agent épaississant ou gélifiant**

Verwendung von hyperverzweigten Polymeren oder Dendrimeren mit einer bestimmten Gruppe als Verdickungsmittel oder Gelierungsmittel

Use of hyperbranched polymers or dendrimers having a particular group as thickening or gelling agent

(84) Etats contractants désignés:
**AT BE CH CY DE DK ES FI FR GB GR IE IT LI LU MC NL PT SE**

(30) Priorité: **19.12.1997 FR 9716176**

(43) Date de publication de la demande:
**27.09.2000 Bulletin 2000/39**

(73) Titulaire: **L'OREAL**
**75008 Paris (FR)**

(72) Inventeurs:
- **MAIGNAN, Jean**
  **F-93290 Tremblay en France (FR)**
- **GENARD, Sylvie**
  **F-75012 Paris (FR)**

(74) Mandataire: **Dodin, Catherine**
**L'OREAL - D.I.P.I.**
**25-29 Quai Aulagnier**
**92600 Asnières (FR)**

(56) Documents cités:
**EP-A- 0 556 871** **WO-A-90/11778**

- **TAKAGISHI TORU AND KLOTZ I.M.: "Macromolecule-Small Molecule Interactions; Introduction of Additional Binding Sites in Polyethyleneimine by Disulfide Cross-linkages" BIOPOLYMERS, vol. 11, 1972, pages 483-491, XP002079069**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 037 938 B1

Printed by Jouve, 75001 PARIS (FR)

## Description

La présente invention concerne de nouveaux composés susceptibles d'être utilisés en cosmétique ou en pharmacie, notamment en dermatologie, et permettant notamment l'obtention de compositions épaissies, voire gélifiées.

Les polymères hyperbranchés et les dendrimères sont bien connus dans l'art antérieur.

Les polymères hyperbranchés sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir : une structure extrêmement ramifiée, autour d'un coeur ; des couches ou générations successives de ramifications ; une couche de chaînes terminales.

Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. On peut faire réagir un groupement terminal T sur le polymère hyperbranché pour obtenir une fonctionnalité particulière en extrémité de chaînes.

Plusieurs polymères hyperbranchés peuvent être associés entre eux, par une liaison covalente ou un autre type de liaison, par l'intermédiaire de leurs groupes terminaux. De tels polymères, dits pontés, entrent dans la définition des polymères hyperbranchés selon la présente invention.

Les dendrimères sont des polymères et oligomères hautement ramifiés ayant une structure chimique bien définie. En règle générale, les dendrimères comprennent un coeur, un nombre déterminé de générations de branches, ou fuseaux, et des groupes terminaux. Les générations de fuseaux sont constituées d'unités structurelles, qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la $N^{ième}$ génération sont les groupes fonctionnels terminaux des fuseaux de la $N^{ième}$ génération ou génération terminale.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération de fuseaux sur la précédente se fait sur les amines $\alpha$ et $\varepsilon$ de la lysine, ce qui conduit à une différence dans la longueur des fuseaux des différentes ramifications.

Les polymères denses en étoiles, ou « dense star polymer », les polymères éclatés en étoile, ou « starburst polymer », les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

On connaît notamment, par la demande de brevet français FR97-04085 au nom de la demanderesse, de nouveaux polymères choisis parmi les polymères hyperbranchés et les dendrimères, et comportant des groupements fonctionnels répondant à la formule suivante :

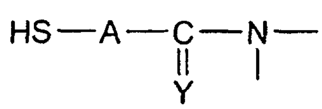

dans laquelle

- * Y représente l'atome d'oxygène ou un groupement NH,

* A représente un groupe alcane di-yle en $C_1$-$C_{12}$, linéaire, ramifié ou cyclique, saturé ou insaturé; ce groupe étant éventuellement interrompu par un ou plusieurs hétéroatomes et/ou substitué par une fonction choisie parmi amino, acylamino, acide carboxylique et ester.

Ces polymères trouvent notamment une application en cosmétique et dermatologie comme agent antioxydant ou agent réducteur.

Or, la demanderesse a constaté avec étonnement que lesdits polymères pouvaient également être utilisés pour permettre la préparation de compositions, notamment cosmétiques ou pharmaceutiques, épaissies, voire gélifiées.

Ainsi, la présente invention a pour objet l'utilisation d'au moins un composé choisi parmi les polymères hyperbranchés et les dendrimères, caractérisé par le fait qu'il comporte au moins un groupement de formule :

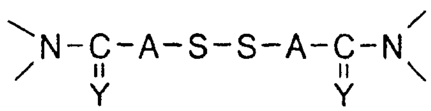

(I)

dans laquelle :

* Y représente l'atome d'oxygène ou un groupe NH, et
* A représente un groupe alcane di-yle en $C_1$-$C_{12}$, linéaire, ramifié ou cyclique, saturé ou insaturé; ce groupe étant éventuellement interrompu par un ou plusieurs hétéroatomes et/ou substitué par une fonction choisie parmi :
  - amino ($-NH_2$);
  - acylamino (-NH-CO-R) dans lequel R représente un groupement alkyle en $C_1$-$C_{10}$ linéaire, ramifié ou cyclique, saturé ou insaturé,
  - acide carboxylique (-COOH),
  - ester (-COOR) dans lequel R représente un groupement alkyle en $C_1$-$C_{10}$ linéaire, ramifié ou cyclique, saturé ou insaturé,

  en tant qu'agent épaississant ou gélifiant, notamment dans une composition cosmétique ou pharmaceutique comprenant en outre un milieu cosmétiquement ou pharmaceutiquement acceptable.

On connaît dans l'art antérieur, un certain nombre de gélifiants ou épaississants usuels, susceptibles d'être utilisés pour adapter la viscosité de compositions notamment cosmétiques. On peut ainsi citer les extraits d'algues tels que l'agar-agar, les carraghénanes, les alginates; les gommes; les extraits de graines, exsudats de plantes ou exsudats de micro-organismes, les dérivés cellulosiques; les extraits de fruits tels que les pectines; les agents gélifiants d'origine animale tels que la gélatine, les caséïnates ou les polymères synthétiques gélifiants hydrosolubles tels que les acides polyacryliques réticulés.

Toutefois, ces gélifiants présentent certains inconvénients. En effet, les gélifiants usuels conduisent à des gels ou à des milieux épaissis dès leur introduction dans ledit milieu. Ils doivent donc nécessairement être introduits dans le milieu avant son application sur le support envisagé.

Or, on a constaté qu'en utilisant les composés selon l'invention ou une composition les comprenant, il était possible de former le gel quand on le souhaitait, et notamment in situ, c'est-à-dire après application de la composition sur le support.

Ainsi, il est possible de former ledit gel avant son conditionnement et de disposer alors d'une composition gélifiée prête à l'application. Dans ce cas, on utilise les composés selon l'invention comme des agents gélifiants usuels.

Toutefois, il est également possible de préparer une composition non gélifiée car non oxydée, de la conditionner telle quelle et de ne réaliser l'oxydation, et donc la gélification, que lors de l'application de la composition sur le support, par exemple par oxydation à l'air libre. Ceci peut notamment être intéressant dans le cas des applications capillaires, de type gel de coiffage ou composition de coloration des cheveux.

Ainsi, l'invention permet de disposer d'un système permettant la gélification de la composition, avant, pendant ou après son application sur le support.

Un autre avantage de l'invention réside dans le fait que dans certains cas, il peut être nécessaire de conserver séparément deux solutions liquides qui doivent être mélangées au moment de l'application, la viscosité du mélange résultant devant être augmentée avant application. C'est le cas notamment de certains produits de coloration des cheveux. Les composés selon l'invention permettent de répondre à ces impératifs.

Les composés objets de l'invention sont donc choisis parmi les polymères hyperbranchés et les dendrimères, et comportent au moins un groupement de formule (I) :

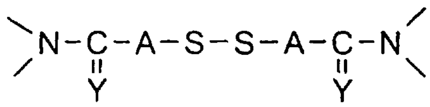

(I)

dans laquelle :

* Y représente O ou NH,
* A représente un groupe alcane di-yle en $C_1$-$C_{12}$, linéaire, ramifié ou cyclique, saturé ou insaturé; ce groupe étant éventuellement interrompu par un ou plusieurs hétéroatomes et/ou substitué par une fonction choisie parmi :
    - amino ($-NH_2$) éventuellement sous forme d'un sel d'un acide minéral ou organique,
    - acylamino (-NH-COR) dans lequel R représente un groupement alkyle en $C_1$-$C_{10}$ linéaire, ramifié ou cyclique, saturé ou insaturé,
    - acide carboxylique (-COOH),
    - ester (-COOR) dans lequel R représente un groupement alkyle en $C_1$-$C_{10}$ linéaire, ramifié ou cyclique, saturé ou insaturé.

De préférence, le composé selon l'invention est choisi parmi les polymères hyperbranchés, et notamment la polyéthylèneimine, comportant au moins un groupement de formule (I).
De préférence, Y représente l'atome d'oxygène.
De préférence, les hétéroatomes sont choisis parmi l'oxygène ou l'azote (O et N).
De préférence, A est un groupement méthylène, éthylène, propylène, méthylpropylène, éthylpropylène, tétraméthylène, pentaméthylène, hexaméthylène, phénylène, phényl di-yle.
Avantageusement, A représente un radical répondant à l'une des formules (a) à (d) suivantes :

(a) $-CHR^1-CHR^2-CHR^3-$

(b) $-CHR'^1-CHR'^2-CHR'^3-CHR'^4-$

(c)

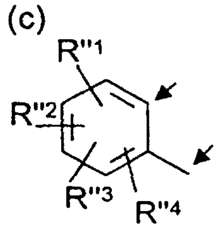

(d) $-(CHR'''^1)_k-(CHR'''^2)-CH(CO_2H)-NH-$

dans lesquelles

* $R^1$, $R^2$, $R^3$, $R'^1$, $R'^2$, $R'^3$ et $R'^4$, $R'''^1$, $R'''^2$, identiques ou différents représentent : l'atome d'hydrogène; un radical alkyle en $C_1$-$C_6$, linéaire, ramifié ou cyclique, saturé ou insaturé; un radical amino ($-NH_2$); un radical acide carboxylique (-COOH); un radical alkylamino en $C_1$-$C_{10}$; un radical acylamino en $C_1$-$C_{10}$.
* $R''^1$, $R''^2$, $R''^3$ et $R''^4$, identiques ou différents représentent : l'atome d'hydrogène; un radical alkyle en $C_1$-$C_4$, linéaire ou ramifié, saturé ou insaturé; les flèches indiquant les positions des substitutions;
* k est un entier, préférentiellement 0 ou 1.

Préférentiellement, A est choisi parmi les groupes suivants :

$$-CH_2-CH(CO_2H)-NH- \; ; \; -(CH_2)_2-(CH_3CONH)CH- \; ; \; -(CH_2)_3- \text{ et}$$

$$-CH_2-CH(NH-CO-CH_3)-$$

Les composés selon l'invention peuvent notamment être obtenus par oxydation des polymères décrits dans la demande FR97-04085 et qui sont choisis parmi les polymères hyperbranchés et les dendrimères, comportant des groupements fonctionnels répondant à la formule (II) :

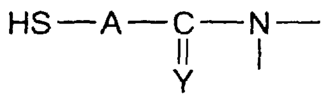

(II)

dans laquelle:

* Y représente O ou NH,
* A représente un groupe alcane di-yle en $C_1$-$C_{12}$, linéaire, ramifié ou cyclique, saturé ou insaturé; ce groupe étant éventuellement interrompu par un ou plusieurs hétéroatomes et/ou substitué par une fonction choisie parmi amino, acylamino, acide carboxylique et ester.

L'oxydation peut être effectuée par tout moyen connu, par exemple à l'air ou à l'aide d'un oxydant usuel tel que le peroxyde d'hydrogène,

L'étape d'oxydation permet la formation de ponts disulfures, intramoléculaires et intermoléculaires, à partir des fonctions thiols, selon le schéma ci-dessous:

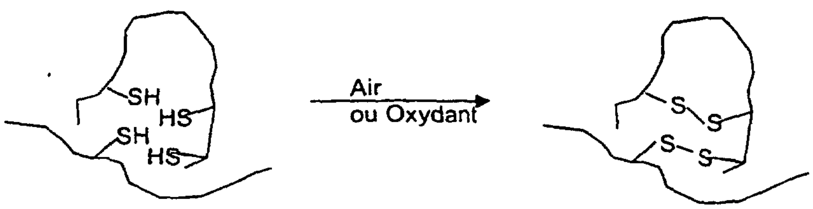


A B

La formation de ponts disulfures entraîne une 'pseudo-réticulation' des composés A de départ, qui se traduit, selon les conditions expérimentales, par un épaississement/une gélification du milieu, dû à la formation des composés B.

On obtient ainsi directement un gel comprenant les composés B.

L'étape d'oxydation est de préférence effectuée en présence d'eau, par exemple en milieu aqueux ou hydroalcoolique.

On peut ainsi obtenir des gels qui peuvent être, de préférence, des gels aqueux ou des gels hydroalcooliques ne renfermant que de l'eau ou un mélange alcool/eau, par exemple éthanol/eau, un ou plusieurs composés B selon l'invention et éventuellement des composés de départ A n'ayant pas réagi, lorsque l'oxydation n'est conduite que partiellement.

D'autre part, on a constaté qu'il était possible d'incorporer dans le milieu aqueux ou hydroalcoolique, des additifs hydrosolubles ou non hydrosolubles, tout en conservant la possibilité d'obtenir une composition de viscosité adéquate.

Parmi les additifs susceptibles d'être incorporés, on peut citer les colorants hydrosolubles tels que la fluorescéïne; des actifs cosmétiques. ou pharmaceutiques hydrosolubles; des produits non hydrosolubles présentant des propriétés optiques telles que phosphorescence ou fluorescence; des pigments; des charges; des filtres solaires; des actifs cosmétiques ou pharmaceutiques non hydrosolubles.

On a de plus constaté que les additifs solides, particules de pigments ou de charges par exemple, étaient parfaitement

dispersés au sein du gel, de manière homogène; lorsque la viscosité du mélange est suffisante, on n'observe ni décantation, ni relargage desdites particules.

On sait que les propriétés des gels obtenus dépendent des conditions d'oxydation, en particulier de la concentration en polymère thiolé de départ, du nombre de fonctions thiols dudit polymère thiolé, de la masse molaire dudit polymère et du pH du milieu aqueuxlhydroalcoolique avant oxydation.

Ainsi, par exemple, pour une masse molaire de poly(éthylèneimine) donnée (masse molaire avant greffage des fonctions thiols), les greffons thiols étant identiques, on a constaté que plus le nombre de greffons est important, plus les composés gélifieront à faible concentration et à pH plus acide.

On obtient donc une solution épaissie ou un gel, qui peut présenter de préférence une viscosité comprise entre $10^{-2}$ et $10^7$ Pa.s, notamment entre 10 et $10^7$, et par exemple entre $10^4$ et $10^6$ Pa.s, et qui peut être utilisé, tel quel, en tant que composition cosmétique ou pharmaceutique, ou incorporé à une composition notamment cosmétique ou pharmaceutique.

Selon les conditions d'oxydation, il est possible que le mélange totalement oxydé demeure liquide avec une très faible augmentation de la viscosité (les disulfures sont solubles dans l'eau à faible concentration). Dans ce cas, on peut concentrer le milieu jusqu'à obtention d'une solution très épaissie ou gélifiée selon l'utilisation envisagée.

Il est également possible d'extraire, totalement ou en partie, l'eau présente dans ledit gel, par exemple par séchage sous vide. Le produit séché ainsi obtenu, qui est généralement hygroscopique, donne à nouveau un gel lorsqu'il est replacé dans l'eau dans les conditions adéquates.

Lorsque l'on ajoute des additifs solides, tels que des particules de pigments par exemple, au milieu avant l'étape d'oxydation et que l'on sèche le gel obtenu, on a constaté que lorsque l'on réhydrate le produit séché par ajout d'eau et/ou d'alcool, on obtient à nouveau un gel présentant les caractéristiques initiales, sans observer notamment de 'relargage' desdites particules solides.

Lorsque le gel comprenant les composés selon l'invention est destiné à être utilisé dans une composition cosmétique ou pharmaceutique, ladite composition comprend par ailleurs un milieu cosmétiquement ou pharmaceutiquement acceptable.

Ladite composition cosmétique ou pharmaceutique peut se présenter sous toutes les formes appropriées pour une application topique, notamment sous forme de gels aqueux ou hydroalcooliques; sous forme d'émulsions eau-dans-huile, huile-dans-eau ou multiple, de consistance liquide plus ou moins épaissie, telles que lait ou crème; de sprays ou de mousse aérosol; de sticks ou bâtons; de solutions ou dispersions liquides.

L'homme du métier sait préparer ces compositions selon les méthodes usuelles, sur la base de ses connaissances générales.

En particulier, ces compositions peuvent contenir des adjuvants habituellement utilisés dans les domaines cosmétique ou pharmaceutique, tels que des huiles, cires ou autres corps gras usuels; des tensioactifs; des agents hydratants; des émollients; des filtres solaires; des actifs hydrophiles ou lipophiles comme des céramides; des agents anti-radicaux libres; des polymères ; des protéines; des bactéricides ; des séquestrants ; des antipelliculaires ; des antioxydants ; des conservateurs ; des agents alcalinisants ou acidifiants ; des parfums; des charges ; des matières colorantes; des actifs cosmétiques ou pharmaceutiques. Les quantités de ces différents adjuvants sont celles classiquement utilisées dans les domaines considérés et sont aisément déterminables par l'homme du métier.

Bien entendu l'homme du métier veillera à choisir ce ou ces éventuels adjuvants complémentaires, et/ou leur quantité, de manière telle que les propriétés avantageuses de la composition selon l'invention ne soient pas, ou substantiellement pas, altérées par l'adjonction envisagée.

Les compositions selon l'invention sont par exemple des lotions, des laits ou des crèmes pour le soin de la peau ou des cheveux; des crèmes, des lotions ou des laits démaquillants; des bases de fond de teint; des lotions, des laits ou des crèmes antisolaires ou après-soleil; des lotions, des laits ou des crèmes de bronzage artificiel; des crèmes ou des mousses de rasage; des lotions après rasage; des compositions d'hygiène corporelle telles que des sticks ou des crèmes déodorants; des shampooings; des produits capillaires pour le maintien de la coiffure ou la mise en forme des cheveux tels que des gels de coiffage; des produits de coloration des cheveux; des rouges à lèvres; des mascaras ou eye-liners éventuellement traitants; des vernis à ongles ou des soins des ongles.

L'invention est illustrée plus en détail dans les exemples suivants.

Les exemples 1 à 8 décrivent la préparation des composés de départ de formule (II).

Les exemples 9 à 16 décrivent la préparation des gels selon l'invention.

Les gels obtenus ont été caractérisés par observation macroscopique et microscopique en lumière polarisée, et par diffraction des rayons X.

Les résultats de ces analyses étaient respectivement : comportement isotrope et absence de cristaux.

Exemple 1 : Polymère branché polyéthylèneimine de poids moléculaire moyen PM=2000 possédant 4 fonctions SH en moyenne par motif

A 50 grammes de solution aqueuse à 50% de polyéthylèneimine de poids moléculaire moyen PM=2000 commercialisée par la société BASF sous la dénomination LUPASOL G35, on ajoute à température ambiante 4,33 ml de γ-thiobutyrolactone (soit 50 mmoles, 4 équivalents molaires calculés par rapport au monomère) sous atmosphère inerte à température ambiante. Le milieu, initialement hétérogène, devient rapidement homogène (environ 30 minutes). Après 4 heures sous agitation, on ne détecte plus de γ-thiobutyrolactone dans le milieu. La phase aqueuse donne une réaction positive après révélation par le nitroprussiate de sodium. On constate ainsi que certaines des fonctions amines primaires initiales sont sous forme de $-NH-CO-(CH_2)_3-SH$.
On dilue cette phase aqueuse par de l'eau qsp 100 ml.
La teneur en matière active de cette phase aqueuse est de 30,11 g/100 ml, soit 0,5 $mol.l^{-1}$ en thiol et 0,125 M en polymère thiolé. Le pH est de 10,3.
Masse molaire du produit synthétisé: 2408,64 $g.mol^{-1}$

Exemple 2 : Polymère branché polyéthylèneimine de poids moléculaire moyen PM=2000 possédant 10 fonctions SH en moyenne par motif

A 30 grammes de solution aqueuse à 50% de polyéthylèneimine de poids moléculaire moyen PM=2000 commercialisée par la société BASF sous la dénomination LUPASOL G35, on ajoute à température ambiante 6,5 ml de γ-thiobutyrolactone (soit 10 équivalents molaires calculés par rapport au monomère) sous atmosphère inerte à température ambiante. Le milieu, initialement hétérogène, devient rapidement homogène (environ 30 minutes). Après 24 heures sous agitation, on ne détecte plus de γ-thiobutyrolactone dans le milieu. La phase aqueuse donne une réaction positive après révélation par le nitroprussiate de sodium.
On dilue cette phase aqueuse par de l'eau qsp 100 ml.
La teneur en matière active de cette phase aqueuse est de 22,66 g/100 ml, soit 0,75 $mol.l^{-1}$ en thiol et 0,075 M en polymère thiolé. Le pH est de 9,6.
Masse molaire du produit synthétisé: 3021,6 $g.mol^{-1}$

Exemple 3 : Polymère branché polyéthylèneimine de poids moléculaire moyen PM=10000 possédant 50 fonctions SH en moyenne par motif

On dilue 11,30 grammes de polyéthylèneimine 99% de poids moléculaire moyen PM=10000 commercialisée par la société POLYSCIENCES par 11,30 ml d'eau, puis on ajoute à température ambiante 4,9 ml de γ-thiobutyrolactone (soit 50 équivalents molaires calculés par rapport au monomère) sous atmosphère inerte à température ambiante. Le milieu, initialement hétérogène, devient rapidement homogène (environ 30 minutes). Après 20 heures sous agitation, on ne détecte plus de γ-thiobutyrolactone dans le milieu. La phase aqueuse donne une réaction positive après révélation par le nitroprussiate de sodium. La masse molaire du produit synthétisé est de 151.08 $g.mol^{-1}$.
On dilue cette phase aqueuse par de l'eau qsp 100 ml et on ajuste le pH à 6,7 par ajout d'une solution aqueuse d'acide chlorhydrique.
On dispose ainsi d'une solution aqueuse homogène à 13,67 g/100 ml en polymère thiolé, soit 0,452 mol/l.

Exemple 4 : Polymère branché polyéthylèneimine de poids moléculaire moyen PM=10000 possédant 20 fonctions SH en moyenne par motif

On dilue 10,15 grammes de polyéthylèneimine 99% de poids moléculaire moyen PM=10000 commercialisée par la société POLYSCIENCES par 10,15 ml d'eau, puis on ajoute à température ambiante 1,76 ml de γ-thiobutyrolactone (soit 20 équivalents molaires calculés par rapport au monomère) sous atmosphère inerte à température ambiante. Le milieu, initialement hétérogène, devient rapidement homogène (environ 30 minutes). Après 20 heures sous agitation, on ne détecte plus de γ-thiobutyrolactone dans le milieu.
On dispose ainsi d'une solution aqueuse de poly(éthylèneimine) thiolée de masse molaire 12043,2 renfermant en moyenne 20 fonctions SH par chaîne de polymère. On dilue cette solution par de l'eau qsp 100ml (formation d'une émulsion stable) et on ajuste le pH à 6 par ajout d'une solution aqueuse d'acide chlorhydrique.
On dispose ainsi d'une solution aqueuse homogène à 10,51 g/100 ml en polymère thiolé soit 0,174 mol/l.

Exemple 5 : Polymère branché polyéthylèneimine de poids moléculaire moyen PM=25000 possédant 50 fonctions SH en moyenne par motif

A 12,28 grammes de solution aqueuse à 56% de polyéthylèneimine de poids moléculaire moyen PM=25000 commercialisée par la société BASF sous la dénomination LUPASOL HF, on ajoute à température ambiante 12,28 g d'eau puis, lorsque le milieu est redevenu homogène, 1,2 ml de γ-thiobutyrolactone (soit 50 équivalents molaires calculés par rapport au monomère) sous atmosphère inerte à température ambiante. Le milieu, initialement hétérogène, devient rapidement homogène (environ 30 minutes). Après 24 heures sous agitation, on ne détecte plus de γ-thiobutyrolactone dans le milieu.
On dispose ainsi d'une solution aqueuse de poly(éthylèneimine) thiolée de masse molaire 30108 renfermant en moyenne 50 fonctions SH par chaîne de polymère.
On dilue 19,60 g de cette solution par de l'eau qsp 25 ml. On dispose ainsi d'une solution aqueuse à 25 g/100 ml en polymère thiolé soit 0,415 mol/l en thiol et 8,30 mmol/l en polymère thiolé. Le pH de cette solution est de 10,65.

Exemple 6 : Polymère branché polyéthylèneimine de poids moléculaire moyen PM=25000 possédant 125 fonctions SH en moyenne par motif

A 22,43 grammes de solution aqueuse à 56% de polyéthylèneimine de poids moléculaire moyen PM=25000 commercialisée par la société BASF sous la dénomination LUPASOL HF, on ajoute à température ambiante 22,43 g d'eau puis 5,44 ml de γ-thiobutyrolactone (soit 125 équivalents molaires calculés par rapport au monomère) sous atmosphère inerte à température ambiante. Le milieu, initialement hétérogène, devient rapidement homogène (environ 30 minutes). Après 24 heures sous agitation, on ne détecte plus de γ-thiobutyrolactone dans le milieu. On dispose ainsi d'une solution aqueuse de poly(éthylèneimine) thiolée de masse molaire 37770, renfermant en moyenne 125 fonctions SH par chaîne de polymère.
On dilue 32,080g de cette solution par de l'eau qsp 50 ml. On dispose ainsi d'une solution aqueuse à 25 g/100 ml en polymère thiolé soit 0,827 mol/l en thiol et 6,619 mmol/l en polymère thiolé. Le pH de cette solution est de 9,96.

Exemple 7 : Préparation du dendrimère thiolé: Dendrimère Starburst (PAMAM) à coeur éthylène diamine de génération 1 possédant 8 fonctions SH en surface

A 5 grammes d'une solution aqueuse à 55,7 g/100 g de dendrimère Starburst (PAMAM) à coeur éthylène diamine de génération 1 (8 fonctions $NH_2$ en surface) dilués par 5 ml d'eau, on ajoute 1,35 ml de γ-thiobutyrolactone (soit 1 équivalent calculé par rapport à l'ensemble des fonctions amines primaires) sous atmosphère inerte à température ambiante. Le milieu hétérogène à l'ajout devient rapidement homogène, après 1 heure.
Après 48 heures sous agitation, on ne détecte que des traces de γ-thiobutyrolactone dans le milieu. On lave trois fois par 10 ml d'éther diéthylique (le milieu est additionné d'éther, agité pendant 10 minutes avant d'être laissé au repos et la phase éthérée est alors séparée). On fait barboter de l'azote dans la phase aqueuse ainsi obtenue pour éliminer toute trace d'éther.
La solution aqueuse ainsi obtenue est analysée par RMN.
On montre ainsi que toutes les fonctions amines primaires initiales sont sous forme $-NH-CO-(CH_2)_3-SH$.
La teneur en matière active de cette phase aqueuse est de 37,76 g/100 g soit 134,42 méq SH/100 g, pH=8.8. Le dendrimère ainsi obtenu (masse molaire 2247,16) est utilisé tel quel en solution aqueuse.

Exemple 8 : Polymère branché poly(éthylèneimine) de poids moléculaire moyen 2000 possédant 11,09 fonctions SH en moyenne par motif

A 50 g de solution aqueuse à 50% de poly(éthylèneimine) de PM=2000 commercialisée par BASF sous la dénomination commerciale LUPASOL G35, on ajoute à température ambiante 12 ml de γ-thiobutyrolactone (soit 11,09 équivalents calculés par rapport au monomère) sous atmosphère inerte à température ambiante (addition légèrement exothermique). Le milieu hétérogène à l'ajout devient rapidement homogène (environ 30 minutes).
Après 16 heures sous agitation, la réaction est terminée (on ne détecte plus de γ-thiobutyrolactone dans le milieu et la phase aqueuse donne une réaction positive après révélation par le nitroprussiate de sodium). On montre ainsi que certaines des fonctions amines initiales sont sous forme $-N-CO-(CH_2)_3-SH$.
On peut diluer cette phase aqueuse par de l'eau qsp 100 ml.
La teneur en matière active de cette phase aqueuse est de 39,16 g/100 ml soit 1,386 $mol.l^{-1}$ en thiol et le pH est de 10,15. Le polymère ainsi obtenu peut être utilisé tel quel en solution aqueuse.
Masse molaire moyenne du produit synthétisé: 3132,95 $g.mol^{-1}$

Exemple 9 : Préparation de gels aqueux à partir de poly(éthylèneimine) thiolée selon l'exemple 1

*a) Gels aqueux "simples"*

A partir de la solution à 30,11 g/100 ml préparée dans l'exemple 1, on réalise diverses solutions par dilution à l'eau et/ou acidification par une solution aqueuse d'acide chlorhydrique.
Leurs caractéristiques sont les suivantes:

| Solution | pH | Concentration en polymère thiolé | Concentration en thiol |
|---|---|---|---|
| 1 | 10,3 | 0,125 M<br>30,11 g/100 ml | 0,500 mol/l |
| 2 | 9,0 | 0,102 M<br>24,56 g/100 ml | 0,408 mol/l |

On oxyde quelques millilitres des solutions 1 et 2 par la quantité théorique d'eau oxygénée à 6% additionnée (soit 0,5 fois le nombre de fonctions thiols à oxyder en disulfure), sous vive agitation.
Les solutions 1 et 2 conduisent instantanément à des gels incolores transparents assez durs.

*b) Gels aqueux renfermant un pigment minéral phosphorescent*

A partir de la solution 1 préparée précédemment, on prépare divers échantillons renfermant du sulfure de zinc dopé par du cuivre et commercialisé sous le nom GREEN LBY 2330 (RN=[68611-70-1]) selon le tableau ci-dessous:

| Solution | % de pigment |
|---|---|
| 3 | 2 |
| 4 | 5 |
| 5 | 10 |

Le % de pigment est calculé en poids par rapport au poids de polymère thiolé.
On agite les échantillons au vortex puis sous ultrasons puis on oxyde quelques millilitres de solution par la quantité théorique d'eau oxygénée à 6% additionnée (soit 0,5 fois le nombre de fonctions thiols à oxyder en disulfure) sous vive agitation (vortex).
Dans ces conditions, les solutions 3, 4 et 5 conduisent instantanément à des gels jaunes très pales assez durs et phosphorescents dans toute la masse du gel. La phosphorescence est d'autant plus intense que la quantité de pigment est importante. Toutes les particules de pigment sont réparties uniformément dans la masse du gel.

Exemple 10 : Préparation de gels aqueux à partir de poly(éthylèneimine) thiolée selon l'exemple 2

*a) Gels aqueux "simples"*

A partir de la solution à 22,66 g/100 ml préparée à l'exemple 2, on réalise diverses solutions par dilution à l'eau et/ou acidification par une solution aqueuse d'acide chlorhydrique.
Leurs caractéristiques sont les suivantes:

| Solution | pH | Concentration en polymère thiolé | Concentration en thiol |
|---|---|---|---|
| 6 | 9,6 | 0,075 M 22,6 g/100 ml | 0,750 mol/l |
| 7 | 8,9 | 69,70 mM 21,06 g/100 ml | 0,697 mol/l |
| 8 | 8,0 | 63,56 mM 19,20 g/100 ml | 0,635 mol/l |
| 9 | 7,0 | 59,71 mM 18,04 g/100 ml | 0.597 mol/l |

(suite)

| Solution | pH | Concentration en polymère thiolé | Concentration en thiol |
|---|---|---|---|
| 10 | 5,95 | 57,16 mM 17,27 g/100 ml | 0,571 mol/l |

On oxyde quelques millilitres des solutions 6 à 10 par la quantité théorique d'eau oxygénée à 6% additionnée sous vive agitation.

Les solutions 6 à 10 conduisent instantanément à des gels.
Les solutions 6 et 7 conduisent à des gels blancs opaques.
Les gels 8, 9 et 10 sont limpides incolores transparents.

Les solutions aqueuses 6 à 10 peuvent être diluées par de l'eau selon les indications du tableau ci-dessous:

| Solution | pH avant dilution | Concentration en polymère thiolé | Concentration en thiol |
|---|---|---|---|
| 11 | 9,6 | 0,03 M<br>9,06 g/100 ml | 0,300 mol/l |
| 12 | 8,9 | 34,85 mM<br>10,53 g/100 ml | 0,349 mol/l |
| 13 | 8,0 | 42,37 mM<br>12,80 g/100 ml | 0,423 mol/l |
| 14 | 7,0 | 39,80 mM<br>12,03 g/100 ml | 0,380 mol/l |
| 14 bis | 7,0 | 33,17 mM<br>10,02 g/100 ml | 0,332 mol/l |
| 15 | 5,95 | 38,10 mM<br>11,51 g/100 ml | 0,381 mol/l |

On oxyde quelques millilitres de solution par la quantité théorique d'eau oxygénée à 6% additionnée sous vive agitation.

La solution 11 conduit instantanément à un gel un peu opaque blanchâtre.
Les solutions 13 et 14 conduisent à des gels limpides incolores transparents.
Les solutions 12 et 15 conduisent à des gels limpides incolores transparents un peu visqueux.

Le gel obtenu avec la solution 14bis a un coefficient de viscosité associé à l'écoulement permanent obtenu par des expériences d'écoulement à l'équilibre de $5.10^5$ Pa.s.
A titre comparatif, la solution 14bis non oxydée mais diluée par un volume d'eau égal au volume d'oxydant ajouté pour former le gel, a un coefficient de viscosité associé à l'écoulement permanent obtenu par des expériences d'écoulement à l'équilibre de $1,2.10^{-3}$ Pa.s.

*b) Gels aqueux renfermant un pigment minéral phosphorescent*

A partir des solutions 8 et 10 préparées précédemment, on prépare divers échantillons renfermant du sulfure de zinc dopé par du cuivre et commercialisé sous le nom GREEN LBY 2330 selon le tableau ci-dessous:

| Solution | pH | Concentration en polymère thiolé | Concentration en thiol (mol/l) | % de pigment |
|---|---|---|---|---|
| 16 | 8,01 | 63,56 mM<br>19,20 g/100 ml | 0,635 | 2 |
| 17 | 8,01 | 63,56 mM<br>19,20 g/100 ml | 0,635 | 5 |
| 18 | 8,01 | 63,56 mM<br>19,20 g/100 ml | 0,635 | 10 |

(suite)

| Solution | pH | Concentration en polymère thiolé | Concentration en thiol (mol/l) | % de pigment |
|---|---|---|---|---|
| 19 | 10,3 | 57,16 mM<br>17,27 g/100 ml | 0,571 | 2 |
| 20 | 10,3 | 57,16 mM<br>17,27 g/100 ml | 0,571 | 5 |
| 21 | 10,3 | 57,16 mM<br>17,27 g/100 ml | 0,571 | 10 |

On agite les échantillons au vortex puis sous ultrasons puis on oxyde quelques millilitres de solution par la quantité théorique d'eau oxygénée à 6% additionnée sous vive agitation.

Les solutions 16, 17 et 18 conduisent instantanément à des gels jaunes très pâles assez durs et phosphorescents dans toute la masse du gel. La phosphorescence est d'autant plus intense que la quantité de pigment est importante. Toutes les particules de pigment sont réparties uniformément dans la masse du gel.

Les solutions 19, 20 et 21 conduisent à des gels après 30 secondes à 2 minutes; le milieu est donc maintenu sous vive agitation alternée avec ultrasons jusqu'à la gélification du milieu. On obtient des gels jaunes très pâles assez durs et phosphorescents dans toute la masse du gel. Toutes les particules de pigment sont réparties uniformément dans la masse du gel.

*c) Gels hydroalcooliques*

A partir de la solution à 22,66 g/100 ml en polymère thiolé préparée selon l'exemple 2, on réalise par acidification par une solution aqueuse d'acide chlorhydrique environ 4N, une solution 19,536 g/100 ml en polymère thiolé à pH 8,97.
A 1 ml de cette solution aqueuse, on ajoute 0,5 ml d'éthanol absolu. On oxyde par 180 µl (microlitres) d'eau oxygénée 1,8M.
On obtient instantanément le gel hydroalcoolique correspondant.

Exemple 11 : Préparation de gels aqueux à partir de poly(éthylèneimine) thiolée selon l'exemple 3

A partir de la solution à 13,67 g/100 ml préparée selon l'exemple 3, on prépare un gel aqueux en oxydant par la quantité théorique d'eau oxygénée à 6% additionnée sous vive agitation (soit 0,5 fois le nombre de fonctions thiols à oxyder en disulfure). On obtient un gel limpide incolore transparent.

Exemple 12 : Préparation de gels aqueux à partir de poly(éthylèneimine) thiolée selon l'exemple 4

A partir de la solution à 10,51 g/100 ml préparée selon l'exemple 4, on prépare un gel aqueux en oxydant par la quantité théorique d'eau oxygénée à 6% additionnée sous vive agitation (soit 0,5 fois le nombre de fonctions thiols à oxyder en disulfure).
On obtient un gel limpide incolore transparent 1 à 2 minutes après addition de l'oxydant.

Exemple 13 : Préparation de gels aqueux à partir de poly(éthylèneimine) thiolée selon l'exemple 5

*a) Gels aqueux "simples"*

A partir de la solution à 25 g/100 ml préparée dans l'exemple 5, on réalise diverses solutions par dilution à l'eau et/ou acidification par une solution aqueuse d'acide chlorhydrique.
Leurs caractéristiques sont les suivantes:

| Solution | pH | Concentration en polymère thiolé | Concentration en thiol (mol/l) |
|---|---|---|---|
| 24 | 10,65 | 8,303 mM<br>25 g/100 ml | 0,415 |

(suite)

| Solution | pH | Concentration en polymère thiolé | Concentration en thiol (mol/l) |
|---|---|---|---|
| 25 | 8,9 | 6,323 mM<br>19,05 g/100 ml | 0,316 |
| 26 | 7,9 | 5,641 mM<br>16,98 g/100 ml | 0,282 |
| 27 | 6,9 | 5,289 mM<br>15,923 g/100 ml | 0,264 |
| 28 | 5,7 | 4,942 mM<br>14,881 g/100 ml | 0,247 |

On oxyde quelques millilitres de solution par la quantité théorique d'eau oxygénée à 6% additionnée sous vive agitation (soit 0,5 fois le nombre de fonctions thiols à oxyder en disulfure).
Les solutions 25 à 28 conduisent à des gels limpides incolores transparents : instantanément pour les exemples 25 et 26, après 30 secondes pour l'exemple 27 et après 2 minutes environ pour l'exemple 28 pour lequel le gel obtenu est encore visqueux.
Les solutions aqueuses 24, 25, 26 et 27 peuvent être diluées par de l'eau selon les indications du tableau ci-dessous:

| Solution | pH avant dilution | Concentration en polymère thiolé | Concentration en thiol (mol/l) |
|---|---|---|---|
| 29 | 10,65 | 2,768 mM<br>8,33 g/100 ml | 0,138 |
| 30 | 8,9 | 4,220 mM<br>12,70 g/100 ml | 0,211 |
| 31 | 7,9 | 3,760 mM<br>11,32 g/100 ml | 0,188 |
| 32 | 6,9 | 4,407 mM<br>13,27 g/100 ml | 0,220 |

On oxyde quelques millilitres de solution par la quantité théorique d'eau oxygénée à 6% additionnée sous vive agitation.
Les solutions 29 et 30 conduisent instantanément à des gels limpides incolores transparents un peu visqueux.
Les solutions 31 et 32 conduisent après 30 secondes à 1 minute à des gels limpides incolores transparents un peu visqueux.

*b) Gels aqueux renfermant des produits solides insolubles dans le milieu*

A partir des solutions 26, 27 et 28 préparées précédemment, on prépare selon le tableau ci-dessous divers échantillons renfermant :

- produit A : pigment phosphorescent (sulfure de zinc dopé par du cuivre et commercialisé sous le nom GREEN LBY 2330)
- produit B : pigment coloré bleu (ULTRAMARINE Blue, CI 77007)
- produit C : pigment coloré jaune (FD&C Yellow n°5 Aluminium Lake, CI 19140:1)
- produit D : colorant non hydrosoluble rouge (D&C Red n°36, CI 12085)
- produit E : produit non hydrosoluble fluorescent (trans, trans-1,4-bis[2-(3,4,5-triméthoxyphényl)vinyl]benzène)

| Solution | pH | Concentration en polymère thiolé | Concentration en thiol (mol/l) | % de pigment |
|---|---|---|---|---|
| 33 | 5,7 | 4,942 mM<br>14,88 g/100 ml | 0,247 | 5% produit A |
| 34 | 7,9 | 5,641 mM<br>16,989/100 ml | 0,282 | 5% produit B |
| 35 | 7,9* | 3,761 mM<br>11,32 g/100 ml | 0,188 | 2% produit C |
| 36 | 6,9 | 5,289 mM<br>15,92 g/100 ml | 0,264 | 1% produit D |
| 37 | 5,7 | 4,942 mM<br>14,88 g/100 ml | 0,247 | 5% produit E |

* pH de la solution avant dilution par ½ volume d'eau

On agite les échantillons au vortex et sous ultrasons puis on oxyde quelques millilitres de solution par la quantité théorique d'eau oxygénée à 6% additionnée sous vive agitation (soit 0,5 fois le nombre de fonctions thiols à oxyder en disulfure).

Les solutions 33 à 37 conduisent instantanément à des gels colorés ou doués de propriétés optiques particulières comme indiqué ci-dessous:

- solution 33 : gel jaune très pâle presque incolore phosphorescent dans toute sa masse. Toutes les particules de pigment sont réparties uniformément dans la masse du gel.
- solution 34 : gel bleu vif intense. Toutes les particules de pigment sont réparties uniformément dans la masse du gel.
- solution 35 : gel jaune vif intense. Toutes les particules de pigment sont réparties uniformément dans la masse du gel.
- solution 36 : gel rouge vif intense. Toutes les particules de colorant sont réparties uniformément dans la masse du gel.
- solution 37 : gel jaune vert 'type fluo', transparent et fluorescent dans le bleu sous irradiation UV à 365 nm. Toutes les particules de produit E sont réparties uniformément dans la masse du gel. Fluorescence dans toute la masse du gel.

*c) Gels aqueux renfermant des colorants hydrosolubles*

A partir des solutions 26, 27 et 28 préparées précédemment, on prépare selon le tableau ci-dessous, divers échantillons renfermant :

- colorant F = fluorescein, sel de sodium (Acid Yellow 73, Color Index 45350)
- colorant G = Rhodamine B (RN=[81-88-9])
- colorant H = Orange G (Acid Orange 10, Color Index 16230)

| Solution | pH | Concentration en polymère thiolé | Concentration en thiol (mol/l) | % de colorant |
|---|---|---|---|---|
| 38 | 7,9* | 4,942 mM<br>14,88 g/100ml | 0,247 | 0,2% produit F |
| 39 | 6,9** | 5,641 mM<br>16,98 g/100ml | 0,282 | 0,1% produit G |
| 40 | 5,7 | 3,761 mM<br>11,32 g/100ml | 0,188 | 0,2% produit H |

* pH de la solution avant dilution par ½ volume de solution aqueuse de colorant

** pH de la solution avant dilution par 1/5 volume de solution aqueuse de colorant

(suite)

| Solution | pH | Concentration en polymère thiolé | Concentration en thiol (mol/l) | % de colorant |
|---|---|---|---|---|
| 41 | 5,7 | 5,289 mM<br>15,92 g/100ml | 0,264 | 0,01% produit F |

On agite les échantillons au vortex et sous ultrasons puis on oxyde quelques millilitres de solution par la quantité théorique d'eau oxygénée à 6% additionnée sous vive agitation (soit 0,5 fois le nombre de fonctions thiols à oxyder en disulfure).

Les solutions 38 à 41 conduisent instantanément à des gels colorés comme indiqué ci-dessous:

- solution 38 : gel jaune vif fluo intense.
- solution 39 : gel rose fuchsia intense.
- solution 40 : gel orange un peu pâle
- solution 41 : gel jaune très pâle.

Exemple 14 : Préparation de gels aqueux à partir de poly(éthylèneimine) thiolée selon l'exemple 6

*a) Gels aqueux "simples"*

A partir de la solution à 25 g/100 ml préparée dans l'exemple 6, on réalise diverses solutions par dilution à l'eau et/ou acidification par une solution aqueuse d'acide chlorhydrique.
Leurs caractéristiques sont les suivantes:

| Solution | pH | Concentration en polymère thiolé | Concentration en thiol (mol/l) |
|---|---|---|---|
| 42 | 8,9 | 5,571 mM<br>21,04 g/100 ml | 0,696 |
| 43 | 8,0 | 5,187 mM<br>19,59 g/100 ml | 0,648 |
| 44 | 6,9 | 4,896 mM<br>18,49 g/100 ml | 0,612 |
| 45 | 5,8 | 4,755 mM<br>17,960 g/100 ml | 0,594 |
| 46 | 5,0 | 4,583 mM<br>17,313 g/100 ml | 0,573 |

On oxyde quelques millilitres de solution par la quantité théorique d'eau oxygénée à 6% additionnée sous vive agitation (soit 0,5 fois le nombre de fonctions thiols à oxyder en disulfure).
Les solutions 43 à 46 conduisent à des gels limpides incolores transparents : instantanément pour les exemples 42 et 43, après environ 10 secondes pour l'exemple 44, après 30 secondes à 1 minute pour l'exemple 45 et après 5 minutes environ pour l'exemple 46.
L'oxydation de la solution 42 conduit à un gel un peu opaque et blanchâtre.

Les solutions aqueuses 42 à 45 peuvent être diluées par de l'eau selon les indications ci-dessous:

| Solution | pH avant dilution | Concentration en polymère thiolé | Concentration en thiol (mol/l) |
|---|---|---|---|
| 47 | 8,9 | 2,786 mM<br>10,52g/100ml | 0,348 |
| 48 | 8,0 | 2,593 mM<br>9,796 g/100 ml | 0,324 |

(suite)

| Solution | pH avant dilution | Concentration en polymère thiolé | Concentration en thiol (mol/l) |
|---|---|---|---|
| 49 | 6,9 | 3,497 mM<br>13,21 g/100 ml | 0,437 |
| 50 | 5,8 | 3,962 mM<br>14,97 g/100 ml | 0,495 |
| 50bis | 8,9 | 3,34 mM<br>12.63 g/100 ml | 0,418 |

On oxyde quelques millilitres de solution par la quantité théorique d'eau oxygénée à 6% additionnée sous vive agitation (soit 0,5 fois le nombre de fonctions thiols à oxyder en disulfure).

Les solutions 48 à 50bis conduisent à des gels limpides incolores transparents: après quelques secondes pour l'exemple 50bis; après 1 minute environ pour les exemples 48 et 49; et après 5 minutes pour l'exemple 50.
La solution 47 après oxydation devient visqueuse en 10-20 secondes.

Le mélange visqueux obtenu avec la solution 47 a un coefficient de viscosité associé à l'écoulement permanent obtenu par des expériences d'écoulement à l'équilibre de $4,5.10^{-2}$ Pa.s.
A titre comparatif, la solution 47 non oxydée mais diluée par un volume d'eau égal au volume d'oxydant ajouté pour l'épaissir, a un coefficient de viscosité associé à l'écoulement permanent obtenu par des expériences d'écoulement à l'équilibre de $1,9.10^{-3}$ Pa.s.
Le gel obtenu avec la solution 50bis a un coefficient de viscosité associé à l'écoulement permanent obtenu par des expériences d'écoulement à l'équilibre d'environ $2.10^{5}$ Pa.s

*b) Gels aqueux renfermant des produits soldes insolubles dans le milieu*

A partir des solutions 42, 43, 44, 45 et 46 préparées précédemment, on prépare divers échantillons renfermant :

- produit A : pigment phosphorescent (sulfure de zinc dopé par du cuivre et commercialisé sous le nom GREEN LBY 2330)
- produit I : pigment coloré bleu (FD&C Blue n°1 Aluminium Lake, Color Index 42090:2)
- produit J : pigment coloré vert (Chromium Hydroxide Green, Color Index 77289)
- produit K : pigment coloré rouge (D&C Red n°7 Calcium Lake)
- produit E : produit non hydrosoluble fluorescent (trans, trans-1,4-bis[2-(3,4,5-triméthoxyphényl)vinyl]benzène)

| Solution | pH | Concentration en polymère thiolé | Concentration en thiol (mol/l) | % de pigment |
|---|---|---|---|---|
| 51 | 8,94* | 2,786 mM<br>10,52g/100 ml | 0,348 | 2% produit I |
| 52 | 8,0* | 2,594 mM<br>9,797 g/100 ml | 0,324 | 1,4% produit J |
| 53 | 6,9** | 4,080 mM<br>15,41 g/100 ml | 0,510 | 3% produit K |
| 54 | 5,8** | 3,962 mM<br>14,966 g/100ml | 0,495 | 3% produit E |
| 55 | 5,0 | 4,584 mM<br>17,31 g/100 ml | 0,573 | 5% produit A |

* pH de la solution avant dilution par 1 volume d'eau

** pH de la solution avant dilution par 1/5 volume d'eau

On agite les échantillons au vortex et sous ultrasons puis on oxyde quelques millilitres de solution par la quantité théorique d'eau oxygénée à 6% additionnée sous vive agitation (soit 0,5 fois le nombre de fonctions thiols à

oxyder en disulfure).
Les solutions 51 à 55 conduisent instantanément à des gels colorés ou doués de propriétés optiques particulières comme indiqué ci-dessous:

- solution 51 : gel bleu intense. Toutes les particules de pigment sont réparties uniformément dans la masse du gel.
- solution 52 : gel vert intense. Toutes les particules de pigment sont réparties uniformément dans la masse du gel.
- solution 53 : gel rouge intense. Toutes les particules de pigment sont réparties uniformément dans la masse du gel
- solution 54 : gel jaune vert 'type fluo', transparent et fluorescent dans le bleu sous irradiation UV à 365 nm. Toutes les particules de pigment sont réparties uniformément dans la masse du gel. Fluorescence dans toute la masse du gel.
- solution 55 : gel jaune très pâle presque incolore phosphorescent dans toute sa masse. Toutes les particules de pigment sont réparties uniformément dans la masse du gel.

*c) Gels aqueux renfermant des colorants hydrosolubles*

A partir des solutions 42, 43, 44, 45 et 46 préparées précédemment, on prépare divers échantillons renfermant :

- colorant F = fluorescein, sel de sodium (Acid Yellow 73, Color Index 45350)
- colorant G = Rhodamine B (RN=[81-88-9] )
- colorant H = Orange G (Acid Orange 10, Color Index 16230)

| Solution | pH | Concentration en polymère thiolé | Concentration en thiol (mol/l) | % de colorant |
|---|---|---|---|---|
| 56 | 8,9* | 2,786 mM<br>10,52 g/100 ml | 0,348 | 0,1% colorant G |
| 57 | 8,0* | 2,594 mM<br>9,80 g/100 ml | 0,324 | 0,2% colorant F |
| 58 | 6,9** | 3,917 mM<br>14,79 g/100 ml | 0,489 | 0,05% colorant G |
| 59 | 5,8*** | 3,962 mM<br>14,97 g/100 ml | 0,495 | 2% colorant F |
| 60 | 5,0 | 4,584 mM<br>17,31 g/100 ml | 0,573 | 1 % colorant H |

* pH de la solution avant dilution par 1 volume de solution aqueuse de colorant
** pH de la solution avant dilution par ½ volume de solution aqueuse de colorant
***pH de la solution avant dilution par 1/5 volume de solution aqueuse de colorant

On agite les échantillons au vortex et sous ultrasons puis on oxyde quelques millilitres de solution par la quantité théorique d'eau oxygénée à 6% additionnée sous vive agitation (soit 0,5 fois le nombre de fonctions thiols à oxyder en disulfure).
Les solutions 56 à 60 conduisent à des gels colorés comme indiqué ci-dessous:

- solution 56 : gel rose fuchsia intense
- solution 57 : gel jaune fluo intense
- solution 58 : gel rose fuchsia intense
- solution 59 : gel jaune fluo intense
- solution 60 : gel orange pâle

Exemple 15 : Préparation de gels aqueux à partir du dendrimère thiolé selon l'exemple 7

A partir de la solution à 37,76 g/100 g préparée selon l'exemple 7, on réalise diverses solutions par dilution à l'eau et/ou acidification par une solution aqueuse d'acide chlorhydrique.
Leurs caractéristiques sont les suivantes:

| Solution | pH | Concentration en dendrimère thiolé | Concentration en thiol (mmol/100g) |
|---|---|---|---|
| 61 | 8,8 | 16,80 mmol/100g<br>37,76 g/100g | 134,42 |
| 62 | 8,8* | 11,20 mmol/100g<br>25,17 g/100g | 89,62 |
| 63 | 7,15 | 15,85 mmol/100g<br>35,62 g/100g | 126,82 |
| 64 | 7,7 | 8,20 mmol/100g<br>18,42 g/100g | 65,57 |

*: avant dilution par l'eau

On oxyde quelques millilitres de solution par la quantité théorique d'eau oxygénée à 6% additionnée sous vive agitation (vortex), soit 0,5 fois le nombre de fonctions thiols à oxyder en disulfure.

Les solutions 61 et 62 conduisent à des gels blancs opaques très durs (instantané pour l'exemple 61; après quelques secondes pour l'exemple 62).

La solution 64 conduit à un gel blanc opaque après quelques minutes.

La solution 63 conduit après quelques minutes à un gel limpide incolore transparent bien rigide.

Exemple 16 : Préparation de gels aqueux à partir de poly(éthylèneimine) thiolée selon l'exemple 8

*a) Gels aqueux "simples"*

A partir de la solution à 39,16 g/100 ml préparée dans l'exemple 8, on réalise diverses solutions par dilution à l'eau et/ou acidification par une solution aqueuse d'acide chlorhydrique.

Leurs caractéristiques sont les suivantes:

| Solution | pH | Concentration en polymère thiolé | Concentration en thiol |
|---|---|---|---|
| 65 | 9,04 | 101,128 mM<br>31,683 g/100 ml | 1 ,121 mol/l |
| 66 | 8,01 | 89,027 mM<br>27,892 g/100 ml | 0,987 mol/l |
| 67 | 7,02 | 82,668 mM<br>25,899 g/100 ml | 0,917 mol/l |
| 68 | 6,04 | 78,121 mM<br>24,475 g/100 ml | 0,866 mol/l |

On oxyde quelques millilitres des solutions 65 à 68 par la quantité théorique d'eau oxygénée à 6% additionnée sous vive agitation (vortex), soit 0,5 fois le nombre de fonctions thiols à oxyder en disulfure.

La solution 65 conduit instantanément à un gel limpide incolore transparent. Les solutions 66, 67 et 68 conduisent à des gels limpides transparents incolores après des temps respectifs de 5-10 secondes, 10-20 secondes et quelques minutes.

Le gel obtenu avec la solution 67 a un coefficient de viscosité associé à l'écoulement permanent obtenu par des expériences d'écoulement à l'équilibre de $3{,}5.10^4$ Pa.s.

A titre comparatif, la solution 67 non oxydée mais diluée par un volume d'eau égal au volume d'oxydant ajouté pour former le gel, a une viscosité de $2.10^{-3}$ Pa.s.

Les solutions 65 à 67 peuvent être diluées par de l'eau selon les indications du tableau ci-dessous:

| Solution | pH avant dilution | Concentration en polymère thiolé | Concentration en thiol |
|---|---|---|---|
| 69 | 9,04 | 21,1218 g/100 ml 67,4185 mM | 0,748 M |
| 70 | 9,04 | 15,0870 g/100 ml 4 8,1561 mM | 0,534 M |
| 71 | 9,04 | 13,7751 g/100 ml 43,9686 mM | 0,488 M |
| 72 | 9,04 | 12,6731 g/100 ml 40,4511 mM | 0,449 M |
| 73 | 8,01 | 13,9458 g/100 ml 44,51353 mM | 0,494 M |
| 74 | 8,01 | 12,678 g/100 ml 40,4668 mM | 0,449 M |
| 75 | 8,01 | 11,6216 g/100 ml 37,0946 mM | 0,411 M |
| 76 | 7,02 | 21,5829 g/100 ml 68,890 mM | 0,764 M |
| 77 | 7,02 | 18,4996 g/100 ml 59,0486 mM | 0,655 M |
| 78 | 7,02 | 17,2663 g/100 ml 55,112 mM | 0,611 M |
| 79 | 7,02 | 16,1871 g/100 ml 51,667 mM | 0,573 M |
| 80 | 7,02 | 14,388 g/100 ml 45,927 mM | 0,509 M |

On oxyde quelques millilitres de solution par la quantité théorique d'eau oxygénée à 6% additionnée sous vive agitation (vortex).

La solution 69 conduit instantanément à un gel limpide incolore transparent.

Les solutions 70 à 80 conduisent à des gels limpides incolores transparents en des temps variant de quelques secondes à quelques minutes.

*b) Gels hydroalcooliques "simples"*

Les solutions 66 et 67 peuvent être diluées par de l'éthanol absolu selon les indications du tableau ci-dessous:

| Solution | pH avant dilution | Dilution par éthanol | Concentration en polymère thiolé | Concentration en thiol |
|---|---|---|---|---|
| 81 | 8,01 | 10 ml sol.66 + 12 ml EtOH | 12,678 g/100 ml 40,4668 mM | 0,4488 M |
| 82 | 7,02 | 10 ml sol. 67 + 5 ml EtOH | 17,2663 g/100 ml 55,112 mM | 0,6112 M |

On oxyde quelques millilitres de solution par la quantité théorique d'eau oxygénée à 6% additionnée sous vive agitation (vortex).

Dans ces conditions, les solutions 81 et 82 conduisent à des gels limpides incolores transparents d'aspects similaires respectivement aux gels obtenus avec les solutions aqueuses 74 et 78.

## Revendications

1. Utilisation d'au moins un composé choisi parmi tes polymères hyperbranchés et les dendrimères, comportant au moins un groupement de formule:

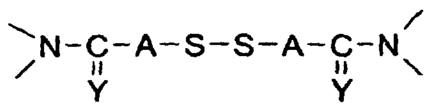

(I)

dans laquelle:

* Y représente l'atome d'oxygène ou un groupe NH, et
* A représente un groupe alcane di-yle en $C_1$-$C_{12}$, linéaire, ramifié ou cyclique, saturé ou insaturé; ce groupe étant éventuellement interrompu par un ou plusieurs hétéroatomes et/ou substitué par une fonction choisie parmi :

  - amino ($-NH_2$);
  - acylamino (-NH-CO-R) dans lequel R représente un groupement alkyle en $C_1$-$C_{10}$ linéaire, ramifié ou cyclique, saturé ou insaturé,
  - acide carboxylique (-COOH),
  - ester (-COOR) dans lequel R représente un groupement alkyle en $C_1$-$C_{10}$ linéaire, ramifié ou cyclique, saturé ou insaturé,

en tant qu'agent épaississant ou gélifiant.

2. Utilisation selon la revendication 1, dans laquelle le polymère hyperbranché est la polyéthylèneimine.

3. Utilisation selon l'une des revendications précédentes, dans lequel Y représente l'atome d'oxygène.

4. Utilisation selon l'une des revendications précédentes, dans lequel A est choisi parmi un groupement méthylène, éthylène, propylène, méthylpropylène, éthylpropylène, tétraméthylène, pentaméthylène, hexaméthylène, phénylène, phényl di-yle ou parmi les groupes suivants :

$-CH_2-CH(CO_2H)-NH-$ , $-(CH_2)_2-(CH_3CONH)CH-$ et $-CH_2-CH(NH-CO-CH_3)-$.

5. Utilisation selon l'une des revendications précédentes, dans une composition cosmétique ou pharmaceutique comprenant en outre un milieu cosmétiquement ou pharmaceutiquement acceptable.

6. Gel aqueux ou hydroalcoolique renfermant de l'eau, ou un mélange alcool/eau, par exemple éthanol/eau, un ou plusieurs composés tels que définis dans l'une des revendications 1 à 5, et éventuellement des composés de départ n'ayant pas réagi.

7. Gel selon la revendication 6 comprenant en outre des additifs hydrosolubles ou non hydrosolubles, et notamment des colorants hydrosolubles tels que la fluorescéïne; des actifs cosmétiques ou pharmaceutiques hydrosolubles; des produits non hydrosolubles présentant des propriétés optiques telles que phosphorescence ou fluorescence; des pigments; des charges; des filtres solaires; des actifs cosmétiques ou pharmaceutiques non hydrosolubles.

8. Composition cosmétique ou pharmaceutique comprenant, dans un milieu cosmétiquement ou pharmaceutiquement acceptable, un gel selon l'une des revendications 6 à 7.

## Patentansprüche

1. Verwendung mindestens einer Verbindung, die ausgewählt ist unter hyperverzweigten Polymeren und Dendrimeren und die mindestens eine Gruppe der Formel

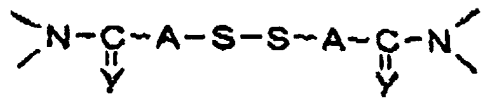 (I)

enthält,
in der bedeuten:

* Y ein Sauerstoffatom oder eine Gruppe NH und
* A eine geradkettige, verzweigte oder cyclische, gesättigte oder ungesättigte $C_{1-12}$-Alkandiyl-Gruppe, die gegebenenfalls durch ein oder mehrere Heteroatome unterbrochen und/ oder durch eine Funktion substituiert ist, die ausgewählt ist unter:

    - Amino ($-NH_2$),
    - Acylamino (-NH-CO-R), wobei R eine geradkettige, verzweigte oder cyclische, gesättigte oder ungesättigte $C_{1-10}$-Alkylgruppe bedeutet,
    - Carbonsäure (-COOH),
    - Ester (-COOR), wobei R eine geradkettige, verzweigte oder cyclische, gesättigte oder ungesättigte $C_{1-10}$-Alkylgruppe bedeutet,

als Verdickungsmittel oder Gelbildner.

**2.** Verwendung nach Anspruch 1, wobei das hyperverzweigte Polymer Polyethylenimin ist.

**3.** Verwendung nach einem der vorhergehenden Ansprüche, wobei Y ein Sauerstoffatom bedeutet.

**4.** Verwendung nach einem der vorhergehenden Ansprüche, wobei A ausgewählt ist unter den Gruppen Methylen, Ethylen, Propylen, Methylpropylen, Ethylpropylen, Tetramethylen, Pentamethylen, Hexamethylen, Phenylen, Phenyldiyl oder folgenden Gruppen:

$$-CH_2-CH(CO_2H)-NH-,\ -(CH_2)_2-(CH_3CONH)CH-\text{ und}$$

$$-CH_2-CH(NH-CO-CH_3)-.$$

**5.** Verwendung nach einem der vorhergehenden Ansprüche in einer kosmetischen oder pharmazeutischen Zusammensetzung, die ferner ein kosmetisch oder pharmazeutisch akzeptables Medium enthält.

**6.** Wässeriges oder wässerig-alkoholisches Gel, das Wasser oder ein Alkohol-Wasser-Gemisch, zum Beispiel Ethanol-Wasser, und eine oder mehrere Verbindungen wie in einem der Ansprüche 1 bis 5 definiert sowie gegebenenfalls nicht umgesetzte Ausgangsverbindungen enthält.

**7.** Gel nach Anspruch 6, das ferner wasserlösliche oder nicht wasserlösliche Additive und insbesondere wasserlösliche Färbemittel, wie Fluorescein; wasserlösliche kosmetische oder pharmazeutische Wirkstoffe; nicht wasserlösliche Produkte, die optische Eigenschaften wie Phosphoreszenz oder Fluoreszenz zeigen; Pigmente; Füllstoffe; Sonnenfilter; nicht wasserlösliche kosmetische oder pharmazeutische Wirkstoffe enthält.

**8.** Kosmetische oder pharmazeutische Zusammensetzung, die in einem kosmetisch oder pharmazeutisch akzeptablen Medium ein Gel nach einem der Ansprüche 6 bis 7 enthält.

## Claims

**1.** Use of at least one compound chosen from hyperbranched polymers and dendrimers, comprising at least one group of formula:

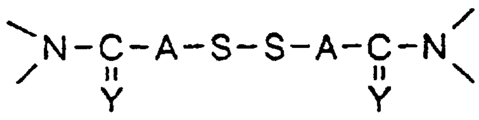

(I)

in which:

* Y represents an oxygen atom or an NH group, and
* A represents a linear, branched or cyclic, saturated or unsaturated $C_1$-$C_{12}$ alkanediyl group, this group optionally being interrupted with one or more hetero atoms and/or substituted with a function chosen from:

  - amino ($-NH_2$),
  - acylamino (-NH-CO-R) in which R represents a linear, branched or cyclic, saturated or unsaturated $C_1$-$C_{10}$ alkyl group,
  - carboxylic acid (-COOH),
  - ester (-COOR) in which R represents a linear, branched or cyclic, saturated or unsaturated $C_1$-$C_{10}$ alkyl group,

as a thickener or gelling agent.

**2.** Use according to Claim 1, in which the hyperbranched polymer is polyethyleneimine.

**3.** Use according to either of the preceding claims, in which Y represents an oxygen atom.

**4.** Use according to one of the preceding claims, in which A is chosen from a methylene, ethylene, propylene, methylpropylene, ethylpropylene, tetramethylene, pentamethylene, hexamethylene, phenylene and phenyl di-yl group, or from the following groups:

$-CH_2-CH(CO_2H)-NH-$, $-(CH_2)_2-(CH_3CONH)CH-$ and $-CH_2-CH(NH-CO-CH_3)-$.

**5.** Use according to one of the preceding claims, in a cosmetic or pharmaceutical composition also comprising a cosmetically or pharmaceutically acceptable medium.

**6.** Aqueous or aqueous-alcoholic gel comprising water or an alcohol/water mixture, ethanol/water for example, one or more compounds as defined in one of Claims 1 to 5, and, optionally, unreacted starting compounds.

**7.** Gel according to Claim 6, also comprising water-soluble or water-insoluble additives, and in particular water-soluble dyes such as fluorescein; water-soluble cosmetic or pharmaceutical active agents; water-insoluble products which have optical properties such as phosphorescence or fluorescence; pigments; fillers; sunscreens; water-insoluble cosmetic or pharmaceutical active agents.

**8.** Cosmetic or pharmaceutical composition comprising, in a cosmetically or pharmaceutically acceptable medium, a gel according to one of Claims 6 to 7.